# EUROPEAN PATENT SPECIFICATION

(11) **EP 2 616 143 B1**
(45) Date of publication and mention of the grant of the patent: **06.05.2015**
(21) Application number: 11769914.0
(22) Date of filing: 12.09.2011
(51) Int. Cl.: A61N 5/06, A61N 2/00, A61N 7/00

(54) **A DEVICE FOR COHERENT AND/OR NON-COHERENT IRRADIATION OF HUMAN BODIES WITH THERAPEUTIC EFFECTS**
VORRICHTUNG FÜR KOHÄRENTE UND/ODER NICHTKOHÄRENTE BESTRAHLUNG VON MENSCHLISCHEN KÖRPERN MIT THERAPEUTISCHEN EFFEKTEN
DISPOSITIF POUR L'IRRADIATION COHÉRENTE ET/OU NON COHÉRENTE DU CORPS HUMAIN AYANT DES EFFETS THÉRAPEUTIQUES

(30) Priority: 13.09.2010 SK 500442010
(43) Date of publication of application: 24.07.2013
(73) Proprietor: Schnugh, Elizabeth Jeanne, Caledon 7230 (ZA)
(72) Inventor: Prinnsloo, Gerhardus Martheunes, deceased (GR)
(74) Representative: Galanopoulos, Sotirios
(86) International application number: PCT/GR2011/000037
(87) International publication number: WO 2012/035363

(56) References cited:
- WO-A1-2007/050144
- WO-A2-2006/125204
- DE-A1- 2 520 108
- DE-A1- 19 642 829
- DE-U1- 29 508 669
- US-A1- 2008 214 968

## Description

### FIELD OF THE INVENTION:

The invention relates to a coherent radiation device with therapeutic effects for the human body, as defined in claim 1.

### BACKGROUND/RECENT STATE OF THE TECHNOLOGY:

Various types of emitters exist within a variety of different electronic devices to stimulate therapeutic benefit for the human body. These radiations are applied externally via physical contact of the therapeutic device with the skin on different parts of the human body. The therapeutic device contacts the body through numerous different types of applicators including brushes, combs, heads, and probes, either handheld or otherwise attached to some other form of control device. Radiation emitters particularly magnetic, optical, and acoustic are modulated at prescribed powers, frequencies and times in order to bring about an overall therapeutic benefit for the human body. Typically, the radiations are applied by bringing the radiation emitters into intimate contact with the skin of the body for prescribed time duration. The therapeutic benefits are determined by the types of radiation emitters used and the specific way in which they are spatially and temporally arranged, applied and controlled. Different electronic and spatial configurations of the emitters lead to different therapeutic benefits to the human body. The quality and extent of the therapeutic benefit is strongly influenced by the collective application of such radiations, and specific knowledge relating to how they should be combined to bring about the described benefits. If the radiations are not applied in a collectively coherent manner which includes the coherency of the actual radiation, the parameters of control of the emitter, the coherence of the collective set of emitters, and the spatial geometry of the emitters, then the therapeutic effect on the human body is significantly degraded, if not completely eliminated.

### SUMMARY:

The invention is defined in claim 1.

The inter-changeable head is of brush-, comb- shape or the inter-changeable head can be of a round or ellipse-shape in cross-section when looking at the radiance emitters, or it can be of tetragonal or triangular shape or in a shape of regular or irregular polygon.

The coherent and/or non-coherent radiant device can have an optic light waveguide attached to the head assigned to each optic radiance emitter. In the handle, there can be a display and/or indicators for device settings to inform the user and/or components to connect the device to external equipment, such as computer and/or device to set and/or control the device and power supply.

In the handle of the coherent radiance device, there can be components to connect the device to external equipment, e.g. a computer with an USB connection and/or wireless connection such as infra-red port and/or Bluetooth. Then, the equipment to charge the device can be - batteries and/or power cord and/or solar cells. The coherent and/or non-coherent radiant device can have an acoustic source integrated in its handle to emit sound signals, e.g. when controlling, switching on/off the device or when starting or finishing the procedure. The handle can also have a visual readout device, e.g. a liquid crystal display, to give the user information regarding the performance, program, feedbacks, operating parameters, and other such useful information in the overall operation of the electronic device. The mobile electronic device is a source of coherent and/or non-coherent, combined electromagnetic, optic and acoustic radiation with defined power, wavelengths, modulation frequency, time and solid geometry of the radiance emitted.

### OVERVIEW OF THE DRAWINGS

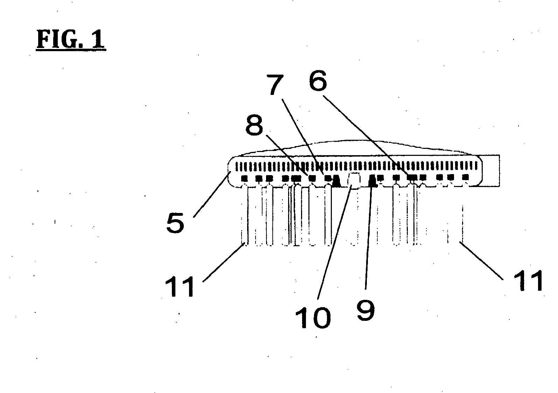

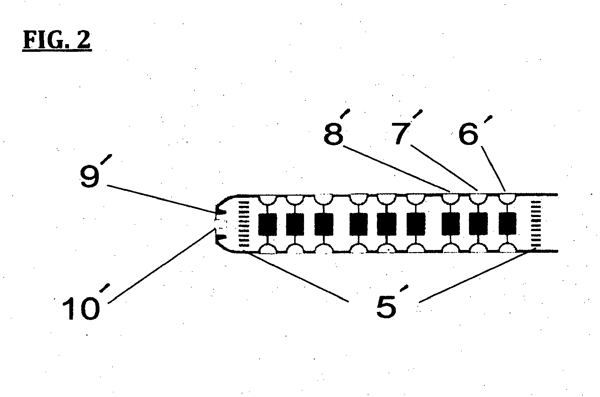

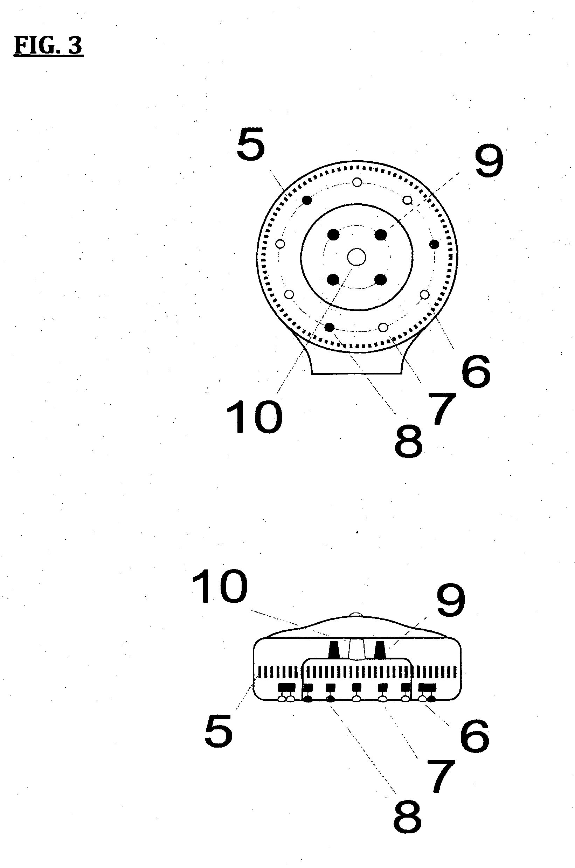

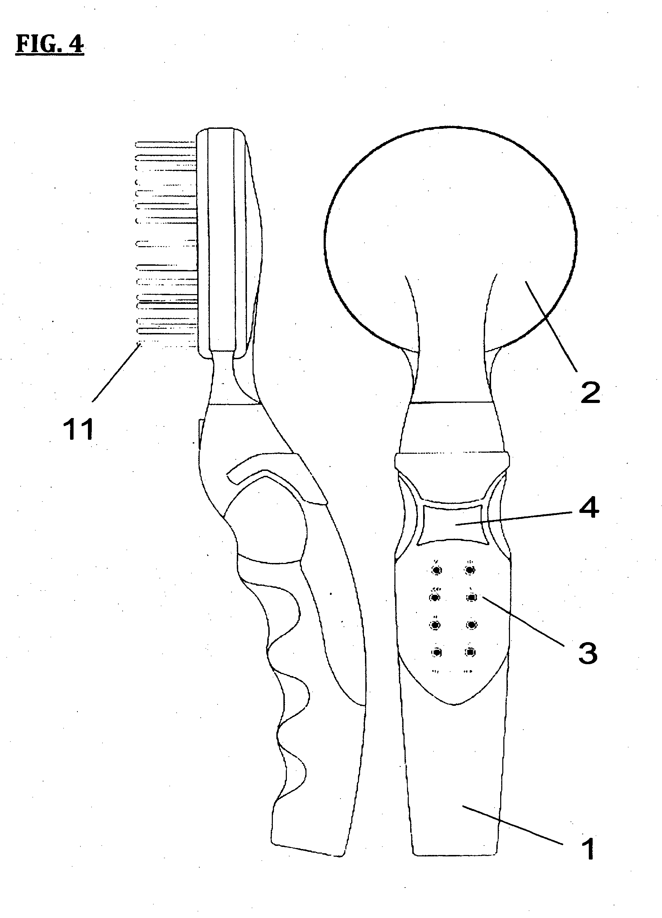

The figure 1 shows the head with optic light waveguides. The figure 2 shows the head of cylindrical shape. The figure 3 shows an illustration of the concentric round-shaped head with. The figure 4 shows the device from two views. All underlined numbers in the text to follow are referring to the numbers shown in all four figures.

### EXAMPLES OF FEASIBILITY

The device for coherent and/or non-coherent radiation of human bodies with therapeutic effects consists of a handle 1 containing the switch button and control components 3 to which an inter-changeable head 2 is attached. In the head 2, there is an electromagnetic field coil, and/or permanent magnets 5 are situated along the external circuit; then there are concentrically and evenly placed red optical radiance emitters 6 from the electromagnetic field coil, and/or permanent magnets 5 toward the geometric center of the head 2; then from concentrically placed red optic radiation emitters 6 toward the center, there are concentrically and evenly placed violet or indigo optic radiation emitters 7; then from the concentrically placed violet or indigo optic radiation emitters 7 toward the center there are concentrically and evenly placed yellow or green optic radiation emitters 8; then from the concentrically placed yellow or green optic radiation emitters 8 toward the center there are concentrically and evenly placed acoustic transducers 9; then, in the geometric center there is an infra-red optic radiation emitter 10. In an alternative device for coherent radiation of human body with therapeutic effects the device consists of a handle 1 containing the switch button and control components 3 to which an inter-changeable head 2 is attached. In the head 2, there is an electromagnetic field coil, and/or permanent magnets 5 are situated along the external circuit; then there are concentrated and equally placed red optical radiance emitters 6 from the electromagnetic field coil, and/or permanent magnets 5 toward the geometric center of the head 2; then from radially and alternately placed red optic radiation emitters 6 there are radially and alternately placed violet or indigo optic radiation emitters 7; then from radially and alternately placed violet or indigo optic radiation emitters 7 there are radially and alternately placed yellow or green optic radiation emitters 8; then from the radially and alternately placed yellow or green optic radiation emitters 8 toward the center, there are concentrically and evenly placed acoustic transducers 9; then, in the geometric center, there is an infra-red optic radiation emitter 10.

In an alternative device for coherent radiation of human body with therapeutic effects that consists of a handle 1 with switch button and control components 3, to which an inter-changeable head is attached, where in the head of cylindrical shape there is an infra-red radiance emitter 10' situated in the center of the front part of the free end; then there are concentrically and equally placed acoustic transducers 9' in the front part from the infra-red radiance emitter 10'; then toward the opposite end there is an electromagnetic field coil, and/or permanent magnets 5' are situated under the external circuit, from where there are concentrically placed yellow or green optic radiance emitters 8' along the circuit toward the opposite end, and then there are violet or indigo optic radiance emitters 7', then the red optic emitters 6', then following the series of radiance emitters 8', 7' and 6', there is an electromagnetic field coil, and/or permanent magnets 5' situated under the external circuit. The inter-changeable head 2 has a shape of a brush or a comb, or the inter-changeable head 2 can be of a round or ellipse shape in cross-section when looking at the radiance emitters 6, 7, and 8, or it can be of tetragonal or triangular shape or in a shape of regular or irregular polygon.

The coherent radiant device can have an optic light waveguide 11 attached to the head 2, assigned to each optic radiance emitter 6, 7, and 8.

In the handle 1, there can be a display 4 and/or indicators 3 for setting the device to inform the user, and/or components to external equipment such as a computer and/or device to set and/or control the device and equipment to charge the device.

In the handle 1 of the coherent radiance device, there can be components to connect the device to an external equipment, e.g. a computer with an USB to connect the device connection and/or wireless connection such as infra-red port and/or Bluetooth.

Then, the equipment for charging the device can be - batteries and/or power cord and/or solar cells.

The coherent and/or non-coherent radiant device can have an acoustic source integrated in its handle 1 to emit sound signals, e.g. when controlling, switching on/off the device or when starting or finishing the procedure.

The handle can also have a visual readout device, e.g. a liquid crystal display, to give the user information regarding the performance, program, feedbacks, operating parameters, and other such useful information in the overall operation of the electronic device.

The portable electronic device is a source of coherent and/or non-coherent, combined electromagnetic, optic and acoustic radiation with defined power, wavelengths, modulation frequency, time and solid geometry of the radiance emitted.

Preferred optical radiation wavelengths for the following is:
a) coherent and/or non-coherent red radiation with the wavelength from 630 nm to 680 nm (nanometer)
b) coherent and/or non-coherent yellow/green radiation with the wavelength from 560 nm to 585 nm
c) coherent and/or non-coherent violet/indigo radiation with the wavelength from 410 nm to 430 nm
d) coherent and/or non-coherent infra-red radiation with the wavelength from 880 nm to 980 nm
e) coherent and/or non-coherent ultra-violet radiation with the wavelength from 280 nm to 360 nm

Combination of radiations with regeneration effects is defined by the order: a, c, b, d or a, c, b, e; and the combination of radiations with revitalization effects is defined by the order: a, b, c, d or a, b, c, e. Preferred acoustic radiation frequencies for the following are:
a) coherent and/or non-coherent acoustic radiation of a frequency between 25KHz and 100KHz for most general therapeutic treatment.
b) coherent and/or non-coherent acoustic radiation of a frequency between 100kHz and 1 MHz for application in specific cases of therapeutic treatment, based on the special detailed knowledge of the invention.

Preferred electromagnetic field radiation is the following:
a) Time varying electromagnetic field patterns both of complex and simple waveform and geometry.

Static magnetic field patterns of both complex and simple electronic and spatial geometry in the strength range 5mT to 100mT at the field centre.

Combination of optical, acoustic and electromagnetic radiations are defined to bring about regeneration effects defined by a combination of optical radiation, and a or b acoustic radiation, and a or b electromagnetic field.

Combination of optical, acoustic and electromagnetic radiations are defined to bring about revitalization effects defined by a combination of optical radiation, and a or b acoustic radiation, and a or b electromagnetic field.

### ANNOTATION

The device for coherent and/or non-coherent radiation of human bodies with therapeutic effects consists of a handle 1 containing the switch button and control components 3 to which an inter-changeable head 2 is attached. In the head 2, there is an electromagnetic field coil, and/or permanent magnets 5 situated along the external circuit; then there are concentrically and equally placed red optical radiance emitters 6 from the electromagnetic field coil, and/or permanent magnets 5 toward the geometric center of the head 2; then from concentrically placed red optic radiation emitters 6 there are concentrically and evenly placed violet or indigo optic radiation emitters 7 toward the center; then from the concentrically placed violet or indigo optic radiation emitters 7 there are concentrically and evenly placed yellow or green optic radiation emitters 8 toward the center; then from the concentrically placed yellow or green optic radiation emitters 8 there are concentrically and evenly placed acoustic transducers 9 toward the center; then in the geometric center there is an infra-red optic radiation emitter 10 placed.

In an alternative device for coherent radiation of human body with therapeutic effects consisting of a handle 1, containing the switch button and control components 3 to which an inter-changeable head 2 is attached. In the head 2, there is an electromagnetic field coil and/or permanent magnets 5 situated along the external circuit; then there are concentrically and equally placed red optical radiance emitters 6 from the electromagnetic field coil and/or permanent magnets 5 toward the geometric center of the head 2; then from radially and alternately placed red optic radiation emitters 6 there are radially and alternately placed violet or indigo optic radiation emitters 7; then from radially and alternately placed violet or indigo optic radiation emitters 7 there are radially and alternately placed yellow or green optic radiation emitters 8; then from the radially and alternately placed yellow or green optic radiation emitters 8 toward the center, there are concentrically and evenly placed acoustic transducers 9; then, in the geometric center, there is an infra-red optic radiation emitter.

In an alternative device for coherent radiation of human body with therapeutic effects consisting of a handle 1 with switch button and control components 3, to which an inter-changeable head is attached, where in the head of cylindrical shape there is an infra-red radiance emitter 10' situated in the center of the front part of the free end; then from the infra-red radiance emitter 10' there are concentrically and equally placed acoustic transducers 9' in the front part; then toward the opposite end there is an electromagnetic field coil and/or permanent magnets 5' are situated under the external circuit, from where there are alternately placed yellow or green optic radiance emitters 8' along the circuit toward the opposite end and then there are violet or indigo optic radiance emitters 7', then the red optic emitters 6', then following the series of radiance emitters 8', 7' and 6' there is the electromagnetic field coil and/or permanent magnets 5' are situated under the external circuit.

## Claims

1. A coherent radiation device with therapeutic effects for the human body, comprising:
a handle (1) with a switch and control components;
an interchangeable head (2) attached to the handle (1);
an infra-red optic radiation emitter (10) in the geometrical centre of the interchangeable head (2);
green or yellow optical radiation emitters (8) concentrically and evenly placed toward the geometrical centre of the interchangeable head (2);
violet or indigo optical radiation emitters (7) concentrically and evenly placed toward the geometrical centre of the interchangeable head (2);
red optical radiation emitters (6) concentrically and evenly placed toward the geometrical centre of the interchangeable head (2);
acoustic transducers (9) concentrically and evenly placed closer to the geometrical centre of the interchangeable head (2);
and an electromagnetic field coil and/or permanent magnets (5) situated along the external circuit inside the interchangeable head (2);
wherein at least said infra-red optic radiation emitter, said acoustic transducers, said green or yellow optical radiation emitters, said violet or indigo optical radiation emitters, and said red optical radiation emitters are modulatable.

2. Device as in claim 1, **characterised by** said interchangeable head (2) having the form of a cylinder or a brush or a comb.

3. Device as in claim 1, **characterised by** an optical light guide (11) being attached to each optical radiation emitter (6, 7, 8) in said interchangeable head (2).

4. Device as in claim 1, **characterised by** a display (4) and/or setting indicators (3) in said handle (1) to inform the user and/or means to connect the device to external equipment, such as computers and/or power supply equipment.

5. Device as in claim 4, **characterised by** means to connect the device with power supply equipment such as a battery and/or solar cells.

6. Device as in claim 4, **characterised by** an integrated acoustic source in said handle (1) for emitting acoustic signals when switching the device on or off or when starting or finishing the procedure.

## Patentansprüche

1. Ein kohärentes Bestrahlungsgerät mit therapeutischen Wirkungen für den menschlichen Körper, bestehend aus:
einem Griff (1) mit Schalt- und Steuerkomponenten;
einem Wechselkopf (2) befestigt am Griff (1);
einem infrarot-optischen Strahler (10) im Geometrischen Zentrum des Wechselkopfes (2);
grünen oder gelben optischen Strahlern (8) konzentrisch und gleichmäßig verteilt zum geometrischen Zentrum des Wechselkopfs (2);
violetten und Indigo optischen Strahlern (7) konzentrisch und gleichmäßig verteilt zum geometrischen Zentrum des Wechselkopfs (2):
roten optischen Strahlern (6) konzentrisch und gleichmäßig verteilt zum geometrischen Zentrum des Wechselkopfs (2);
akustischen Wandlern (9) konzentrisch und gleichmäßig verteilt näher zum geometrischen Zentrum des Wechselkopfs (2);
und einer elektromagnetischen Feldspule und/oder Permanentmagneten (5), die sich längs des äußeren Stromkreises im Wechselkopf befinden (2);
dabei sind mindestens der genannte Infrarot-optische Strahler, die genannten akustischen Wandler, genannten grünen und gelben optischen Strahler, genannten violetten oder indigo optischen Strahler und genannten optische roten Strahler moduliert.

2. Gerät wie in der Behauptung 1 ist **gekennzeichnet durch** den genannten Wechselkopf (2) in der Form von einem Zylinder, Bürste oder Kamm.

3. Gerät wie in der Behauptung 1 ist **gekennzeichnet durch** einen optischen Lichtleiter (11), der zu jedem optischen Strahler (6, 7, 8) an dem genannten Wechselkopf (2) befestigt ist.

4. Gerät wie in der Behauptung 1 ist **gekennzeichnet durch** eine Anzeige (4) und/oder Einstellungsindikatoren (3) im genanntem Griff (1) **durch** die der Benutzer informiert wird und/oder das ein Mittel zum Anschließen des Geräts zu einem externen Gerät, wie z.B. Rechner und/oder zu Stromversorgungsanlagen, dient.

5. Gerät wie in der Behauptung 4 ist gekennzeichnet als ein Mittel zum Anschließen des Geräts zu Stromversorgungsanlagen, wie z.B. Batterien und/oder Solarzellen, dient.

6. Gerät wie in der Behauptung 4 ist gekennzeichnet als eine integrierte akustische Quelle am genannten Griff (1) für das Senden von akustischen Signalen beim Ein- und Ausschalten des Geräts oder beim Start oder Beenden von Prozeduren.

## Revendications

1. Dispositif de rayonnement cohérent avec les effets thérapeutiques sur le corps humain comprenant:
une manche (1) avec un interrupteur et des pièces de commande ;
une tête interchangeable (2) fixée à la manche (1) ;
un émetteur de rayonnement optique infra-rouge (10) au centre géométrique de la tête interchangeable (2) ;
des émetteurs optiques de rayonnement (8) verts ou jaunes concentriquement et uniformément placés vers le centre géométrique de la tête interchangeable (2) ;
des émetteurs de rayonnement optiques (7) violets ou indigo concentriquement et uniformément placés vers le centre géométrique de la tête interchangeable (2) ;
des émetteurs de rayonnement optiques (6) rouges concentriquement et uniformément placés vers le centre géométrique de la tête interchangeable (2) ;
des transducteurs acoustiques (9) concentriquenent et uniformément placés plus proche du centre géométrique de la tête interchangeable (2) ;
et une bobine de champ électromagnétique et / ou des aimants permanents (5) situés dans le circuit extérieur à l'intérieur de la tête interchangeable (2) ;
dans lequel au moins ledit émetteur de rayonnement optique infra-rouge, lesdites transducteurs acoustiques, lesdits émetteurs de rayonnement optique verts ou jaunes, lesdites émetteurs de rayonnement optique violets ou indigo et lesdites émetteurs de rayonnement optique rouges sont modulables.

2. Dispositif selon la revendication 1 **caractérisé par** ladite tête interchangeable (2) ayant la forme d'un cylindre ou d'une brosse ou d'un peigne.

3. Dispositif selon la revendication 1 **caractérisé par** un guide de lumière optique (11) attaché à chaque émetteur de rayonnement optique (6, 7, 8) dans ladite tête interchangeable (2).

4. Dispositif selon la revendication 1 **caractérisé par** un écran (4) et / ou des indicateurs de réglage (3) dans ladite manche (1) pour informer l'utilisateur et / ou les moyens pour la connexion de l'appareil à un équipement externe, tels que les ordinateurs et / ou les équipements d'alimentation électrique.

5. Dispositif selon la revendication 4 **caractérisé par** des moyens pour la connexion de l'appareil à un équipement d'alimentation électrique, tels qu'une batterie et / ou des piles solaires.

6. Dispositif selon la revendication 4 **caractérisé par** une source acoustique intégrée dans ladite manche (1) pour émettre des signaux acoustiques lors de l'activation ou déactivation du dispositif ou lors du démarrage ou de fin de la procédure.
